# EUROPEAN PATENT SPECIFICATION

(11) **EP 2 519 268 B1**
(45) Date of publication and mention of the grant of the patent: **17.04.2019**
(21) Application number: 10800872.3
(22) Date of filing: 30.12.2010
(51) Int. Cl.: A61L 27/04, A61L 29/02, A61F 2/06, A61F 2/24, C21C 7/00, C21C 7/10, C21D 8/00, C22C 38/04, C22C 38/06, A61F 2/86, A61C 7/20, C22C 38/00

(54) **ENDOPROSTHESIS CONTAINING MULTI-PHASE FERROUS STEEL**
ENDOPROTHESE MIT MEHRPHASIGEM EISENSTAHL
ENDOPROTHÈSE CONTENANT UN ACIER FERREUX MULTIPHASIQUE

(30) Priority: 29.12.2010 US 981102; 31.12.2009 US 291497 P
(43) Date of publication of application: 07.11.2012
(73) Proprietor: W.L. Gore & Associates, Inc., Newark DE 19714 (US)
(72) Inventor: BLANZY, Jeffrey, S., Flagstaff AZ 86001 (US)
(74) Representative: HGF Limited
(86) International application number: PCT/US2010/062460
(87) International publication number: WO 2011/082280

(56) References cited:
- US-A1- 2002 138 134
- US-A1- 2008 119 943
- SYRETT, DAVIS: "In Vivo Evaluation of a High-Strength, High-Ductility Stainless Steel for use in Surgical Implants", JOURNAL OF BIOMEDICAL MATERIALS RESEARCH, vol. 13, 1 January 1979 (1979-01-01), pages 543-556, XP002634364, DOI: 10.1002/jbm.820130403

## Description

### FIELD OF THE INVENTION

The present invention relates to the field of endoprostheses, and particularly to the field of diametrically expandable endoprostheses.

### BACKGROUND OF THE INVENTION

Various types of metallic materials have been used in implanted medical devices in the past. Type 316L or a 316LVM stainless steel, cobalt-chromium alloys, commercially pure titanium, and titanium alloys are typical metals used for implantable devices. The environment and method of implantation dictates the use of certain raw materials with specific biocompatibility and material properties. These materials typically possess the necessary physical properties such as tensile strength, fatigue resistance, elastic recoil and yield strength for specific applications.

It is often desirable, to form these metallic materials into complex shapes (including diametrically expandable shapes) such as artificial heart valves, stents, and filters. These types of applications would typically require a metallic material with strength properties close to that of 316L or a 316LVM stainless steel as well as an elastic recoil similar to 316L or a 316LVM. There are often applications that require that these complex shapes be expanded in size (e.g., via a balloon) to conform or comply with certain geometry, be that anatomical or device-driven geometry. In these applications, the metallic material selected would have a relatively low yield strength to allow ease of expansion. The intended environment of implantation of some of these devices (e.g., coronary stents) typically requires a metallic material with a relatively high strength.

Device geometry, method of delivery and environment often force the choice of a metallic material that compromises in one of the four important physical property areas: tensile strength, fatigue resistance, elastic recoil or yield strength. For these reasons, the choice of a metallic material for a particular application is often challenging and compromising.

In relation to other advanced high-strength steels, multiple phase steels (i.e., multi-phase steel) exhibit better ductility at a given strength level. In an example of one multiple phase steel, dual phase steel, the enhanced formability stems from the combination of ferrite and martensite phases present in the raw material. Dual phase steel has a high work hardening rate that enable it to behave in a stable manner during a stamping for forming process. Dual phase steel may be purchased from a supplier such as AK Steel (West Chester, OH. 45069).

In another example of multiple phase steels, TRIP (Transformation Induced Plasticity) steel, enhanced formability comes from the transformation of retained austenite (ductile, high temperature phase of iron) to martensite (tough, non-equilibrium phase) during plastic deformation. Enhanced formability also stems from a high work hardening rate, which enable the metal to behave in a stable way during a stamping or forming process. Because of this increased formability, TRIP steel may be used to produce more complex shapes than other high strength steels. TRIP steel may be purchased from suppliers such as US Steel (Pittsburgh, PA) or ArcelorMittal (Brazil).

TRIP steel containing 4% Mo has been evaluated against type 316L or a 316LVM stainless steel and cast Vitallium alloy as a potential material for use as an implantable material for orthopaedic applications. Results from in vivo evaluation of TRIP steel versus 316L stainless steel in these applications showed that TRIP steel was susceptible to stress-corrosion cracking and much more susceptible to crevice corrosion. Document US 2002/138134 A1 discloses medical implants made of duplex stainless steel which have been thermally treated. These implants (stents, wires) can generate heat by themselves when submitted to a magnetic radiation.

### SUMMARY OF THE INVENTION

A first embodiment provides an endoprosthesis (i.e., a prosthesis that is placed internally within a body) comprised of a multi-phase (multiple phase) ferrous stainless steel as defined in the appended claims. Multi-phase ferrous stainless steel (also referred to as Advanced High Strength Steel, or AHSS) is defined as any ferrous steel with more than one phase (e.g., austenite, ferrite, banite or martensite) present in the microstructure. Multi-phase ferrous stainless steel will encompass such steels as dual phase, complex phase (more than two phases present in the microstructure), duplex, TRIP, TWIP (Twinning Induced Plasticity) and Q&P (Quenched and Partitioned).

A second embodiment provides a method of making an endoprosthesis comprising the steps of forming (e.g., stamping, wire winding or laser cutting) a multiphase steel material such as TRIP stainless steel into a desired shape, forming the desired shape into a tubular form and crimping (e.g..affixing/securing) said tubular form onto a balloon-based endovascular delivery system, delivering said desired shape to an area of treatment, and expanding said desired shape at the area of treatment by inflation of the balloon.

### BRIEF DESCRIPTION OF THE DRAWINGS

Figure 1 illustrates a stress-strain curve for L605, 316L or a 316LVM, dual phase steel and TRIP steel.
Figure 2 is a graph showing change in recoil of L605, 316L or a 316LVM
Figures 3A and 3B are perspective views of one embodiment of multi-phase ferrous stainless steel endoprosthesis before and after diametrical expansion.
Figure 4 shows a longitudinal cross sectional view of a multi-phase ferrous stainless steel endoprosthesis mounted on and diametrically expanded by a typical balloon delivery system.
Figure 5 is a perspective view of one embodiment of an endovascular delivered balloon expandable multi-phase ferrous stainless steel heart valve.
Figure 6 shows a perspective view of one embodiment of a surgically implantable heart valve containing multi-phase ferrous stainless steel.
Figure 7 shows a side view of an implantable filter device.
Figure 8 shows a side view of an alternative balloon expandable stent made from a multi-phase ferrous steel.
Figure 9 shows a view of the stent of Figure 8 as made from a sheet of multi-phase ferrous steel.
Figure 10 shows a stent-graft utilizing multiple balloon expandable stents of the type described by Figure 8.

### DETAILED DESCRIPTION OF THE INVENTION

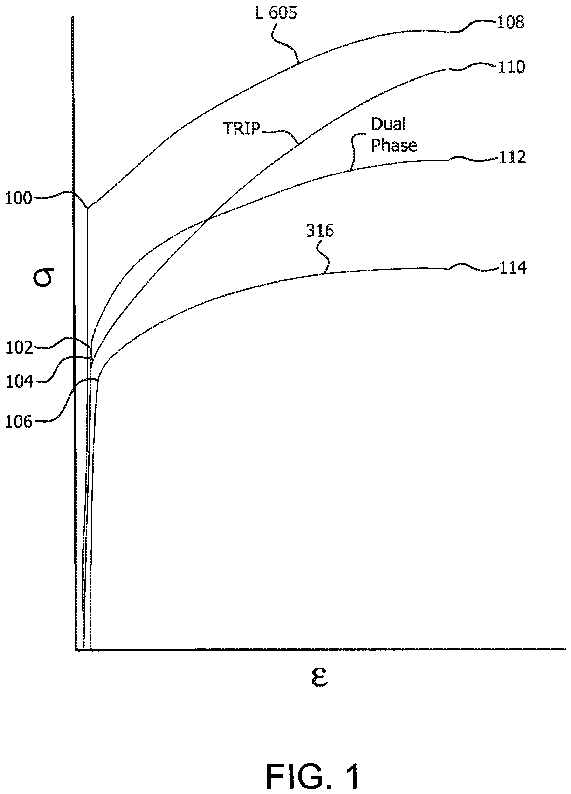

Figure 1 illustrates a stress-strain curve comparing the typical properties of a L605 cobalt chromium alloy, a dual phase steel, a TRIP steel, and a 316L or a 316LVM stainless steel. As shown in the figure, L605 has a relatively high yield strength (YS) 100 and a high ultimate tensile strength (UTS) 108 while 316L or a 316LVM has a lower yield strength 106 and a lower ultimate tensile strength 114. Dual phase steel (102) and TRIP steel (104) have a yield strength that is typically lower than that of L605 (100) which enhances formability and ease of expansion. It is noteworthy that the ultimate tensile strength of dual phase steel (102) and TRIP steel (104) are higher than the ultimate tensile strength of 316L or 316LVM (114).

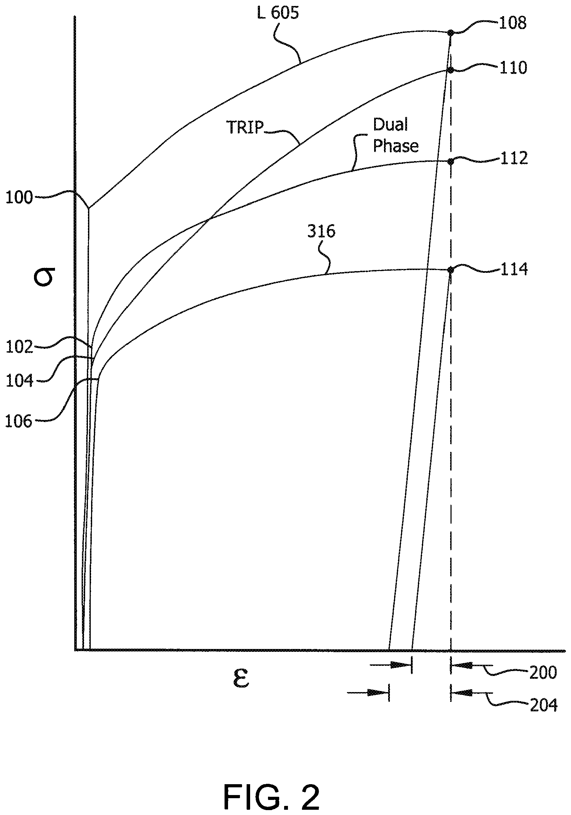

Figure 2 shows stress-strain curves with change in recoil indicated for typical L605 and 316L or a 316LVM steels used in endoprostheses. The change in recoil for a 316L or a 316LVM steel is shown as strain amount 200 while the change in recoil for a L605 is shown as strain amount 204. The strain amount 204 shows a typical recoil amount for a high modulus, high yield strength metal such as L605. Strain amount 200 shows a typical recoil amount for a low modulus, low yield strength metal such as 316L or a 316LVM. Change in recoil in a dual phase or TRIP steel would fall between the two values. The advantages of a material for use as an endoprosthesis that exhibits the relatively small amount of recoil as that of a 316L or a 316LVM while maintaining the high ultimate tensile strength and a high modulus of L605 would be advantageous.

MRI (Magnetic Resonance Imaging) compatibility is an important property in any metal chosen for an implantable prosthesis. Duplex Stainless steels present a fine microstructure of paramagnetic austenite and ferromagnetic ferrite with a microstructure ratio that typically is around 50% for each phase. Stainless steels like 316 LVM are considered to be MRI compatible because they have a microstructure that is 100% austenite and thus paramagnetic. Materials like plain carbon steels have a ferrite microstructure and are ferromagnetic. Ferromagnetic materials not considered MRI-safe or MRI-compatible due to the fact that they are strongly influenced by magnetic fields. It has been shown that the volume fraction of ferrite can be reduced in Duplex stainless steel through heat treatment. For example, Duplex steel samples have been heat treated in a vacuum furnace to a temperature of 1300°C and then slowly cooled (inside of the furnace) to 1000°C and next removed from the furnace and air cooled to room temperature. This processing technique decomposed the ferrite volume fraction in the microstructure from 50% to about 11% without the formation of any secondary brittle sigma phase.
Such a sample was then tested using thermomagnetic analysis and was shown to have a very poor ferromagnetic signal due to the low content of ferrite. The thermomagnetic curve was considered to be typical of a paramagnetic material.

A first embodiment provides an endoprosthesis comprised of a multi-phase ferrous steel as defined in Table 1. These multi-phase ferrous endoprostheses may be fabricated by known means (some of which are described below) as used for such devices made from conventional materials. An example of such an endoprosthesis would be that of a coronary stent. Typically, coronary stents are produced using either a cobalt chromium alloy for post deployment strength, or a 316L or a 316LVM stainless steel for conformability, trackability, minimal elastic recoil, and ease of formability. Stents made from any of these metals are frequently produced with complex geometrical designs. The designs are typically formed using a variety of methods. Some designs are formed from metallic wire into a generally tubular shape. More complex designs are either cut from a thin flat sheet of metal and then bent to form a tube from the cut design or cut directly from a thin tubular form. Either method may then be diametrically compacted to allow the stent to be secured to a balloon catheter. Cutting of the pattern may take place by a variety of means commonly known in the art including but not limited to electrical discharge machining, chemical etching, stamping, or laser cutting. Due to the unique mechanical stresses placed on a coronary stent during manufacture and during delivery of the stent to the desired implant site, the metallic material most widely used is 316L or a 316LVM stainless steel. Typically, these pre-cut metals used to make commercially available coronary stents are excessively thick due to the mechanical demands placed on the deployed device. The properties of multi-phase ferrous steels would allow these same devices to be made with thinner walls while still offering good strength properties.

Coronary stents are typically delivered percutaneously to the desired implant site by attachment onto the outside of a balloon catheter. The catheter carrying the stent is maneuvered through the vasculature of a patient, which is often complex and tortuous. If the metallic material chosen for the stent possesses high strength characteristics, such as cobalt chromium alloy, its ability to successfully navigate the tortuous anatomy may be compromised and upon deployment it will exhibit an inherent recoil. Given the environment of implantation and mechanical needs of a coronary stent, the use of a multiple phase steel would more ideally meet the demands placed on the stent design during formation, delivery and post deployment and rectify many of the aforementioned compromises.

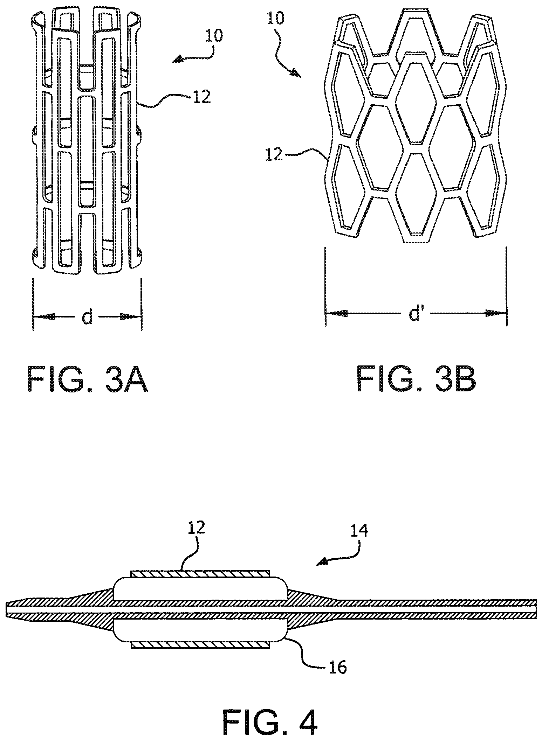

Figures 3A and 3B are perspective views of one embodiment of multi-phase ferrous stainless endoprostheses 10 (e.g., stent 12) before and after diametrical expansion, with the diameter difference indicated in the respective figures as d and d'.

Figure 4 shows a longitudinal cross sectional view of a multi-phase ferrous stainless steel endoprosthesis 10 (e.g., stent 12) mounted on and diametrically expanded by an inflated catheter balloon 16, all part of a typical balloon delivery system 14.

A further example of an endoprosthesis would be that of a renal stent which may be formed in the same manner and basic shapes as the coronary stent described above. Most renal stents are constructed with two distinct sections, the ostial lesion region and the distal section, to comply with different anatomical demands. The ostial region of a renal stent has high radial strength requirements and is usually constructed with a thicker wall and more longitudinal connectors. The distal portion of a renal stent is desired to be more flexible than the ostial region and is usually constructed with a thinner wall and fewer connectors. The entire stent is desired to be low profile for optimal trackability, accurate placement and must be designed to inflate quickly and easily so as not to block the arteries for any length of time. These conflicting design requirements dictate a compromise in material choice. Most available renal stents are made from 316L or a 316LVM stainless steel. As with other stents and frames discussed above, 316L or a 316LVM allows greater trackability, formability and minimal elastic recoil. In order to achieve these performance goals, the stent must be designed in two distinct sections which increases manufacturing difficulty.

If a multiple phase steel were used, the design could be made homogenous without compromising the needed attributes of high radial strength, flexibility, trackability, and ease of balloon expansion. The design could be made with a thin wall and fewer connectors throughout.

Various other types of diametrically expandable stents may benefit from the use of multi-phase ferrous steels for their manufacture. These can include stents for peripheral, carotid, brain (neural), biliary, hepatic, aortic and thoracic applications. Again, these may be fabricated by known methods. Any or all of these types of stent devices may be provided as stent-grafts wherein the stent frame is given a partial or entire covering (on either the outer, inner or both surfaces of the stent) of a prosthetic graft material such as dacron or ePTFE (expanded polytetrafluoroethylene).

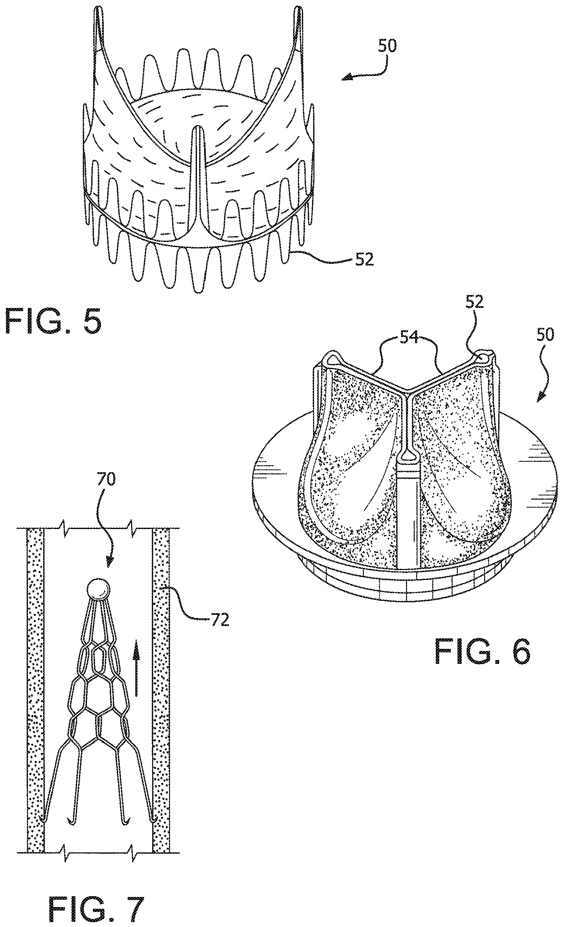

A further example of an endoprosthesis would be that of a transcatheter-delivered prosthetic heart valve 50 like those shown in Figures 5 and 6. Transcatheter delivered heart valves are typically made from a frame of medical grade stainless steel chosen for the material's formability, trackability characteristics, and minimal elastic recoil. It is also possible to make them from a cobalt nickel or cobalt chromium alloy chosen for the material's mechanical strength. These transcatheter delivered heart valves are deployed directly to the sight of an existing malfunctioning heart valve therefore they take up space that could be otherwise utilized for blood flow. Methods for forming a heart valve frame 52 are similar to those used to form a stent and have been discussed previously. Designs for a frame 52 are often ring-shaped and formed of rows of zig-zag or sinusoidal type undulations (Figure 5) with longitudinal connectors between the rows. Alternately, they may be formed of diamond shaped elements connected together to form rings. Many other shapes may be envisioned for the frame 52 of a transcatheter-delivered heart valve 50.

Frame 52 has attached a valve material. Materials for a valve 54 could be homografts (donor graft), autografts (typically via the Ross procedure), heterograft or xenograft (animal tissue grafts from most commonly, bovine or porcine donors), or of any biocompatible material such as PTFE (polytetrafluoroethylene) or ePTFE (expanded polytetrafluoroethylene). These materials may be attached to the frame with a variety of methods commonly know in the art such as suturing directly to the frame or suturing to a skirt of another material (e.g., Dacron® or polyester) and then suturing or chemical bonding to the frame.

These heart valves 50 are deployed by a balloon catheter in two methods: transapically or transfemorally; the most common route of delivery is transfemorally. This method of delivery demands the ability of the device attached to a balloon catheter to be flexible enough to track through a considerable length of potentially tortuous anatomy. This trackability demand often dictates the use of a medical grade stainless steel for the heart valve frame.

If the typical medical grade stainless steel is chosen for the frame 52 of a heart valve 50, frame 52 must be somewhat thicker than if a stronger material were chosen such as a cobalt chromium alloy or a cobalt nickel alloy. The mechanical stresses imparted to a valve frame 52 are considerable in the environment of a beating heart. The use of a cobalt alloy would hinder the typical method of delivery of the device as well as render the deployment less accurate. In other words, a thinner frame is desirable to facilitate blood flow and device delivery but the frame must be sufficiently strong to hold up under the mechanical stresses imparted by a beating heart. Multiple phase steel would meet the unique mechanical demands of transcatheter delivered heart valves.

Surgical staples or sternal closure devices may also be beneficially made from multi-phase ferrous steels. Staples are often used to close bowel, lung and skin wounds.

Implantable filters 70 such as shown by Figure 7 as implanted in a blood vessel 72 may also be effectively manufactured from the multi-phase ferrous stainless steels described herein. These filters can include inferior vena cava filters and embolic filters. Filters for these applications are often made to be diametrically expandable to allow for insertion into a body conduit for subsequent expansion at a desired site.

Other medical applications for duplex stainless steels would be in the arena of medical leads. Medical pacing leads have an electrical connector component which has a compressible portion that expands to accept an inserted lead and then contracts or is crimped around the lead to provide both an electrical and a spring-like mechanical connection to the lead. Ideally, this crimp connection would be very thin and flexible until the crimp is made but of sufficient strength to withstand the high tensile forces imparted to the lead during implant and explantation. A material such as duplex stainless steel would be an optimal choice for such an application.

Guidewires may also be manufactured from multi-phase ferrous steels.

Orthodontic prosthetics, in particular arch wires, are another application for multi-phase ferrous metals. Arch wires must be able to be formed with very little force but must exert a constant force (chosen by the dentist to be sufficient to cause tooth movement but not painful) over a strain range of up to 5%. This constant force must be maintained without much recoil. Since the load may be applied mechanically, a material that is strong and easily formable would be desirable.

Additional processing steps may be added to the fabrication of any of the above-mentioned devices. For example, a fatigue-life improvement step could be added after forming a device shape. This step may involve pre-straining selected portions of the formed device, electropolishing the formed device, or media blasting the formed device to impart compressive residual stresses at the surface of the metal. If the multi-phase ferrous metal were to be supplied with an annealed surface, this processing step could be performed prior to device formation. A further processing step could also be added to improve bonding strength for coating or cover adhesion. This step is similar to that for fatigue life improvement but results in improving bonding life. As with fatigue life improvement, this step could be performed either prior to or following device formation depending on the raw material provided.

Endoprostheses as described above may be provided with coatings of a variety of types of bioactive substances (therapeutic agents), such as blood thinners or antibiotics. These may be bonded to such devices by a variety of known methods appropriate to the desired bioactive agent. They may also be optionally coated with various polymers, optionally containing therapeutic agents, as desired for specific applications. Suitable coatings may include fluoropolymers such as fluorinated ethylene propylene (FEP), polytetrafluoroethylene (PTFE), ePTFE and copolymers of tetrafluoroethylene and polyalkylvinylethers such as polyalkylmethylether (TFE/PMVE).

### Example 1

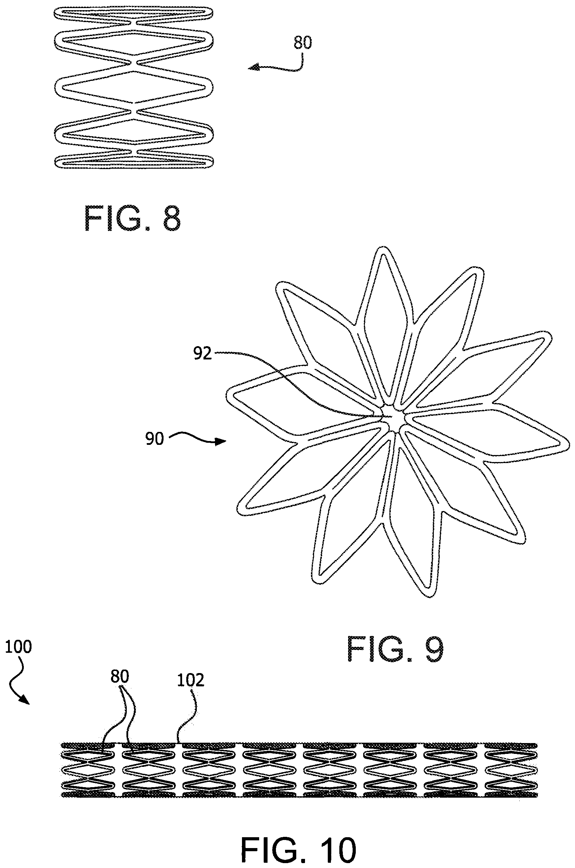

Figure 8 shows a balloon expandable tubular endoprosthesis 80 of an exemplary type that may be made from multi-phase ferrous steel. For clarity, only the side of the device closest to the viewer is shown in Figure 8, with the back side of the tubular form (furthest from the viewer) omitted, as such a device would generally appear to a viewer if a mandrel or other cylindrical form were inserted into the interior of the tubular form of the device. Figure 8 illustrates the endoprosthesis 80 as it would appear following partial diametrical expansion with a catheter balloon. A device of this type was manufactured using Duplex Grade S2205 (available from Sandmeyer Steel Co., Philadelphia PA) in the form of a steel plate of 6.35mm thickness. The steel plate as received had the following properties:
UTS of 845 MPa
0.2% YS of 644 MPa
Elongation (%) of 29
Volume fraction of austenite of 56.4% and ferrite of 43.6%.

The volume fraction of austenite and ferrite was measured using x-ray diffraction techniques with a copper source. The measurements were made in the center of the plate where the plate was cross-sectioned.

The steel plate was heat treated at 1300°C and furnace cooled to 1000°C. After reaching 1000°C the plate was cooled in ambient air to room temperature. The steel plate following heat treatment had the following properties:
UTS of 781 MPa
0.2% YS of 485 MPa
Elongation (%) of 34
Modulus of 216 GPa
Volume fraction of austenite of 41.4% and ferrite of 58.6%.

Tensi|e testing was done in accordance with ASTM E8. Tensile samples from the heat-treated stainless steel plate were machined into threaded tensile bars. Laser cut tensile strips were cut from the 316LVM and L605 tubing and also tested in tension. The mechanical properties of the 316 LVM were as follows:
UTS of 661 MPa
0.2% YS of 340 MPa
Elongation (%) of 53
Modulus of 126 GPa

The mechanical properties of the comparative L605 samples tested were as follows:
UTS of 1079 MPa
0.2% YS of 567 MPa
Elongation (%) of 56
Modulus of 235 GPa

This testing showed that the heat-treated Duplex stainless steel has a modulus of elasticity, yield strength, and ultimate tensile strength that are between the two alloys while the total elongation is less then 316LVM and L605.

After heat treatment, hypotubes were wire EDM (Electrical Discharge Machine) machined (Mitsubishi Wire EDM, model FA205) from the steel plate. These hypotubes had an outer diameter of 4.57mm and a wall thickness of 0.254mm. Since the EDM tubes were too small in length to be laser cut, stainless steel tube extenders were made and press fitted into the ends of the hypotubes. Stent rings of the type shown in Figure 8 were then laser cut from the hypotubes; diameter and wall thickness were not affected. Laser cutting was performed at the expanded diameter of the endoprosthesis (i.e., the diameter the device would have following typical balloon expansion of the device), so that the appearance was generally as shown by Figure 8. Laser cut stent rings of the same type and the same dimensions were made from 316LVM alloys. The Duplex rings and the 316LVM alloy rings underwent a simulated crimp to 1.5mm. The rings were then radially expanded using a tapered mandrel to 10mm and put into the Blockwise J-crimper (Model RJAT, Blockwise Engineering LLC, Phoenix AZ). The J-crimper was mounted into an Instron tensile tester (Model 5564, Instron Corp., Norwood MA) and the rings were individually placed into the mechanical iris. The rings were then individually diametrically crushed in the iris to an intermediate size (1.65mm outside diameter) and the strength of the rings was determined with the Instron Bluehill software. The Duplex ring was shown to be about 20% stronger than the 316LVM rings.

Recoil of the Duplex laser cut rings was measured using the following process. Endoprosthesis rings (stent rings) of the type described above were fabricated of the heat-treated Duplex S2205 steel, and of both 316LVM, and L605 similar to that described previously. These rings were diametrically expanded using a tapered stainless steel mandrel having a maximum diameter 12.80mm cylindrical end portion. The rings were expanded to an inner diameter of 12.80mm and then removed from the tapered mandrel. These 12.80mm diameter was considered functionally relevant for stent rings of this design. Following diametrical expansion and removal from the tapered mandrel, the inner diameter of each ring was measured using a Nikon vision system (Model VMR 320 type 3). The diameter of each ring was measured at ten different locations evenly spaced around the inner diameter of the stent and averaged. These measurements demonstrated a recoil of 0.051 mm in the 316LVM stent ring, 0.152mm in the Duplex steel stent ring, and 0.279mm in the L605 stent ring. These data indicate that the L605 ring has a higher degree of elastic recoil as compared to the heat-treated Duplex ring as is therefore less formable.

In addition to being machined from a billet as generally described above, stent rings of the type shown by Figure 8 may also be machined from sheet materials. A machined pattern 90 for such a stent is shown in Figure 9. Following machining of the sheet, the resulting planar form 90 is then shaped into a tube using a tapered mandrel. The small diameter of the mandrel must be capable of being inserted into the center opening 92 of planar from 90. The mandrel should have a maximum diameter equal to the intended inside diameter of the partially expanded stent form; this maximum diameter would include an equal diameter adjacent cylindrical section. Inserting the small end of the mandrel into the center opening 92 of planar form 90 and pushing the mandrel entirely through the planar form 90 results in a tubular form 80 as shown in Figure 8.

Multiple stent rings were made as described above made from the heat-treated Duplex S2205 steel. Eight rings 80 were joined to the outer surface of a graft material such as the ePTFE tube 102 to create a stent-graft 100 as shown in Figure 10. The manufacture of stent-grafts of this type is described in US Published Patent Application No. 2008/0119943. The resulting balloon expandable stent-graft 100 of approximately 40mm length could be loaded onto a balloon catheter for subsequent delivery into the vasculature of a patient and subsequent balloon expansion. It is appreciated that the stent-graft 100 shown in Figure 10 is exemplary only and that many forms of stent-grafts incorporating stents made of multi-phase ferrous steel are possible. It is likewise appreciated that the stent may be joined to the outer surface of the graft material, the luminal surface of the graft material or may be sandwiched between inner and outer layers of graft material. Further, the graft material may incorporate perforations if desired for particular applications such as biliary therapy.

While the Duplex S2205 stainless steel alloy, particularly when heat-treated as described above, has been shown to offer good strength capabilities and good forming capabilities for the manufacture of balloon expandable endoprostheses, it is believed that even better alloys are possible for medical devices and particularly expandable endoprostheses. Table 1 shows the composition of one such allow. It is appreciated that small deviations from this composition may also offer some improvement over the Duplex S2205 alloy.

**Table 1**

| Element | Wt% |
|---|---|
| C | 0.03 max |
| Mn | 2.0 max |
| Si | 0.75 max |
| Cr | 16.0-18.0 |
| Ni | 6.0-8.0 |
| Mo | 0.6-.09 |
| P | 0.03 max |
| S | 0.02 max |
| N | 0.2-0.25 |
| Fe | Balance |
| W | 0.8-12 |

In addition to being directed to the embodiments described above and claimed below, the present invention is further directed to embodiments having different combination of the features described above and claimed below. As such, the invention is also directed to other embodiments having any other possible combination of the dependent features claimed below.

## Claims

1. An endoprosthesis comprising a multiple phase ferrous steel comprising 16.0- 18.0 wt% chromium; 6.0-8.0 wt% nickel; 0.8-1.2 wt% tungsten; 0.6- 0.9 wt% molybdenum; 0.2-0.25 wt% nitrogen; at most 2.0 wt% manganese; at most 0.75 wt% silicon; at most 0.03 wt% phosphorus; at most 0.02 wt% sulphur and at most 0.03 wt% carbon, the balance being iron.

2. An endoprosthesis according to claim 1 wherein the multiple phase steel is,
(i) a dual phase steel, or
(ii) a complex phase steel, or
(iii) a duplex steel, or
(iv) a transformation induced plasticity steel, or
(v) a twinning induced plasticity steel, or
(vi) a quenched and partitioned steel.

3. An endoprosthesis according to claim 1 comprising:
(i) a diametrically expandable device, or
(ii) a diametrically expandable device comprising a balloon expandable stent, and optionally comprising a stent-graft, or
(iii) a filter device, or
(iv) an orthodontic wire, or
(v) comprising an electrical crimp connector.

4. An endoprosthesis as claimed in claim 1 further comprising PTFE.

5. An endoprosthesis as claimed in claim 1 further comprising a bioactive substance.

6. A method of making an endoprosthesis comprising the steps of forming a tubular form from a multiple phase ferrous steel comprising 16.0- 18.0 wt% chromium; 6.0-8.0 wt% nickel; 0.8-1.2 wt% tungsten; 0.6- 0.9 wt% molybdenum; 0.2-0.25 wt% nitrogen; at most 2.0 wt% manganese; at most 0.75 wt% silicon; at most 0.03 wt% phosphorus; at most 0.02 wt% sulphur and at most 0.03 wt% carbon, the balance being iron.,
crimping said tubular form onto a balloon based endovascular delivery system, for delivery of said desired shape to an area treatment to allow to be expanded at the area of treatment.

7. The method according to claim 6, wherein the tubular form is formed from a flat sheet of the stainless steel alloy, or from a wire of the stainless steel alloy.

8. The method of claim 6 further comprising the steps of impacting the formed device with a media to impart compressive residual stresses at the surface of the alloy.

9. The method of claim 6, further comprising the step of electropolishing the formed device prior to crimping said tubular form onto a balloon based endovascular delivery system.

## Patentansprüche

1. Endoprothese, die einen mehrphasigen Eisenstahl umfasst, der 16,0-18,0 Gew.-% Chrom; 6,0-8,0 Gew.-% Nickel; 0,8-1,2 Gew.-% Wolfram; 0,6-0,9 Gew.-% Molybdän; 0,2-0,25 Gew.-% Stickstoff; höchstens 2,0 Gew.-% Mangan; höchstens 0,75 Gew.-% Silizium; höchstens 0,03 Gew.-% Phosphor; höchstens 0,02 Gew.-% Schwefel und höchstens 0,03 Gew.-% Kohlenstoff umfasst, wobei der Rest aus Eisen besteht.

2. Endoprothese nach Anspruch 1, wobei der mehrphasige Stahl Folgendes ist:
(i) ein Dualphasenstahl, oder
(ii) ein Komplexphasenstahl, oder
(iii) ein Duplexstahl, oder
(iv) ein Transformation-Induced-Plasticity-Stahl, oder
(v) ein Twinning-Induced-Plasticity-Stahl, oder
(vi) ein abgeschreckter und unterteilter Stahl.

3. Endoprothese nach Anspruch 1, die Folgendes umfasst:
(i) eine diametral ausdehnbare Vorrichtung, oder
(ii) eine diametral ausdehnbare Vorrichtung, die einen ausdehnbaren Ballonstent umfasst, und gegebenenfalls einen Stentgraft umfasst, oder
(iii) eine Filtervorrichtung, oder
(iv) einen Bogendraht, oder
(v) umfassend einen elektrischen Crimpverbinder.

4. Endoprothese nach Anspruch 1, ferner umfassend PTFE.

5. Endoprothese nach Anspruch 1, ferner umfassend eine bioaktive Substanz.

6. Verfahren zur Herstellung einer Endoprothese, das die folgenden Schritte umfasst: Bilden einer Röhrenform aus einem mehrphasigen Eisenstahl, der 16,0-18,0 Gew.-% Chrom; 6,0-8,0 Gew.-% Nickel; 0,8-1,2 Gew.-% Wolfram; 0,6-0,9 Gew.-% Molybdän; 0,2-0,25 Gew.-% Stickstoff; höchstens 2,0 Gew.-% Mangan; höchstens 0,75 Gew.-% Silizium; höchstens 0,03 Gew.-% Phosphor; höchstens 0,02 Gew.-% Schwefel und höchstens 0,03 Gew.-% Kohlenstoff umfasst, wobei der Rest aus Eisen besteht,
Aufquetschen der Röhrenform auf ein ballonbasiertes endovaskuläres Einbringungssystem zum Einbringen der gewünschten Form in einen Behandlungsbereich, um ihr zu ermöglichen, in dem Behandlungsbereich ausgedehnt zu werden.

7. Verfahren nach Anspruch 6, wobei die Röhrenform aus einem flachen Bogen der Edelstahllegierung oder aus einem Draht der Edelstahllegierung gebildet wird.

8. Verfahren nach Anspruch 6, ferner umfassend die folgenden Schritte: Anstoßen der gebildeten Vorrichtung mit einem Medium, um eine Restdruckspannung auf die Fläche der Legierung zu übertragen.

9. Verfahren nach Anspruch 6, ferner umfassend den Schritt des Elektropolierens der gebildeten Vorrichtung vor dem Aufquetschen der Röhrenform auf ein ballonbasiertes endovaskuläres Einbringungssystem.

## Revendications

1. Endoprothèse comprenant un acier ferreux multiphasique comprenant de 16,0 à 18,0 % en poids de chrome ; de 6,0 à 8,0 % en poids de nickel ; de 0,8 à 1,2 % en poids de tungstène ; de 0,6 à 0,9 % en poids de molybdène ; de 0,2 à 0,25 % en poids d'azote ; au plus 2,0 % en poids de manganèse ; au plus 0,75 % en poids de silicium ; au plus 0,03 % en poids de phosphore ; au plus 0,02 % en poids de soufre et au plus 0,03 % en poids de carbone, le reste étant du fer.

2. Endoprothèse selon la revendication 1 dans laquelle l'acier multiphasique est,
(i) un acier double-phase, ou
(ii) un acier à phase complexe, ou
(iii) un acier duplex, ou
(iv) un acier à plasticité induite par transformation, ou
(v) un acier à plasticité induite par maclage, ou
(vi) un acier trempé et partitionné.

3. Endoprothèse selon la revendication 1, comprenant :
(i) un dispositif diamétralement expansible, ou
(ii) un dispositif diamétralement expansible comprenant un stent expansible à ballonnet, et facultativement comprenant un stent couvert, ou
(iii) un dispositif de filtration, ou
(iv) un fil orthodontique, ou
(v) comprenant un connecteur à sertir électrique.

4. Endoprothèse selon la revendication 1 comprenant en outre du PTFE.

5. Endoprothèse selon la revendication 1 comprenant en outre une substance bioactive.

6. Procédé de fabrication d'une endoprothèse comprenant les étapes de formation d'une forme tubulaire à partir d'un acier ferreux multiphasique comprenant de 16,0 à 18,0 % en poids de chrome ; de 6,0 à 8,0 % en poids de nickel ; de 0,8 à 1,2 % en poids de tungstène ; de 0,6 à 0,9 % en poids de molybdène ; de 0,2 à 0,25 % en poids d'azote ; au plus 2,0 % en poids de manganèse ; au plus 0,75 % en poids de silicium ; au plus 0,03 % en poids de phosphore ; au plus 0,02 % en poids de soufre et au plus 0,03 % en poids de carbone, le reste étant du fer,
le sertissage de ladite forme tubulaire sur un système de délivrance endovasculaire à base de ballonnet, pour la délivrance de ladite forme souhaitée vers une zone de traitement pour permettre qu'elle soit déployée au niveau de la zone de traitement.

7. Procédé selon la revendication 6, dans lequel la forme tubulaire est formée à partir d'une feuille plate de l'alliage d'acier inoxydable, ou à partir d'un fil de l'alliage d'acier inoxydable.

8. Procédé selon la revendication 6 comprenant en outre les étapes d'impact du dispositif formé avec un support pour transmettre des contraintes de compression résiduelles à la surface de l'alliage.

9. Procédé selon la revendication 6, comprenant en outre l'étape d'électropolissage du dispositif formé avant le sertissage de ladite forme tubulaire sur un système de délivrance endovasculaire à base de ballonnet.
